(19) Europäisches Patentamt
European Patent Office
Office européen des brevets

(11) Publication number: 0 054 414
B1

(12) # EUROPEAN PATENT SPECIFICATION

(45) Date of publication of patent specification: 20.03.85

(21) Application number: 81305829.4

(22) Date of filing: 10.12.81

(51) Int. Cl.$^4$: G 03 C 1/42, G 03 C 1/02, G 03 C 1/06, G 03 C 5/30, C 07 D 249/12

(54) **Photographic material containing a silver halide stabilizer precursor compound.**

(30) Priority: 12.12.80 US 215783

(43) Date of publication of application: 23.06.82 Bulletin 82/25

(45) Publication of the grant of the patent: 20.03.85 Bulletin 85/12

(84) Designated Contracting States: BE DE FR GB

(56) References cited:
US-A-3 708 304
US-A-4 138 265
US-A-4 170 480

(73) Proprietor: EASTMAN KODAK COMPANY
343 State Street
Rochester New York 14650 (US)

(72) Inventor: Altland, Henry Wolf
Kodak Park
Rochester New York (US)
Inventor: Shiao, Daniel Da-Fong
Kodak Park
Rochester New York (US)

(74) Representative: Pepper, John Herbert et al
KODAK LIMITED Patent Department P.O. Box 114 190 High Holborn
London WC1V 7EA (GB)

EP 0 054 414 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to 1,2,4-triazolium-3-thiolate silver halide stabilizer precursor compounds and their use in heat developable and heat stabilizable photographic silver halide material.

Although use of stabilizer percursor compounds in heat developable and heat stabilizable photographic materials is known from U.S. Patents 3,708,304 and 4,012,260, for instance, a problem frequently exists with use of such compounds in that they can cause undesired sensitometric changes in photographic silver halide materials prior to exposure and processing. Moreover, the known stabilizer compounds lack sufficient water solubility to permit their effective use in photographic silver halide materials to provide water-soluble, light-insensitive silver (I) complexes upon exposure and heating of a heat developable and heat stabilizable photographic silver halide material.

The present invention provides a photographic material which contains a silver halide stabilizer precursor compound which is stable prior to exposure and processing but which is capable of releasing a stabilizing agent for form a water soluble, light-insensitive silver (I) complex upon exposure and processing of the photographic material.

According to the invention, a developed and stabilized silver image is provided in a heat developable and heat stabilizable photographic silver halide material comprising a support having thereon a layer which contains, or adjacent layers which together contain:

(a) photographic silver halide, preferably as a photographic silver halide gelatino emulsion;
(b) a photographic silver halide developing agent;
(c) a concentration of a thermal base releasing compound sufficient to activate the developing agent upon heating the photographic material;
(d) a stabilizing concentration of a heterocyclic sulfur-containing silver halide stabilizer precursor, and
(e) a binder.

The heterocyclic sulfur-containing silver halide stabilizer precursor, according to the invention, comprises a novel 1,2,4-triazolium-3-thiolate silver halide stabilizer precursor in which the sulfur atom is blocked by an appropriate blocking group which is released at processing temperature. The silver halide stabilizer precursor provides, upon heating and after development of an exposed photographic silver halide material, a stabilized silver image.

Any 1,2,4-triazolium-3-thiolate silver halide stabilizer can form the basis of a precursor according to the invention. Combinations of such stabilizer precursors are useful. Preferred 1,2,4-triazolium-3-thiolate stabilizer precursors are represented by the formula:

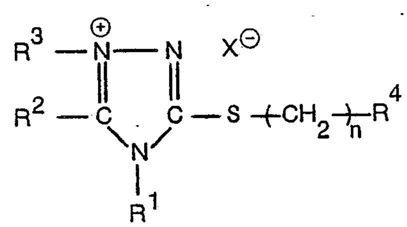

wherein:

$R^1$ is substituted or unsubstituted alkyl containing 1 to 18 carbon atoms, such as methyl, ethyl, propyl, butyl, decyl, benzyl, 2-phenethyl, octadecyl and alkoxyalkyl, preferably containing 2 to 12 carbon atoms, such as methoxymethyl, 2-methoxyethyl, 3-methoxypropyl and 4-methoxybutyl; $NR^5R^6$ wherein $R^5$ and $R^6$ can be hydrogen, substituted or unsubstituted alkyl of from 1 to 18 carbon atoms or substituted or unsubstituted aryl of from 6 to 20 carbon atoms, with the proviso that when $R^5$ is alkyl, $R^6$ is also alkyl; alkenyl containing 3 to 18 carbon atoms, such as allyl, 2-butenyl or crotonyl; cycloalkyl containing 3 to 12 carbon atoms, such as cyclohexyl and cycloheptyl; substituted or unsubstituted aryl containing 6 to 20 carbon atoms, such as phenyl, 4-tolyl and α-naphthyl;

$R^2$ is substituted or unsubstituted alkyl containing 1 to 9 carbon atoms, such as methyl, ethyl, propyl, butyl and pentyl; or aryl containing 6 to 12 carbon atoms, such as phenyl and α-naphthyl;

$R^3$ is substituted or unsubstituted alkyl containing 1 to 18 carbon atoms, such as methyl, ethyl, propyl, butyl, pentyl, decyl and octadecyl; or substituted or unsubstituted aryl containing 6 to 12 carbon atoms, such as phenyl and α-naphthyl; or cycloalkyl of from 3 to 12 carbon atoms; and

$R^4$ is substituted or unsubstituted alkyl containing 1 to 18 carbon atoms, such as methyl, ethyl, propyl, decyl and octadecyl; substituted or unsubstituted aryl containing 6 to 12 carbon atoms, such as phenyl and α-naphthyl; carboxyaryl containing 7 to 13 carbon atoms, including carboxyphenyl, and carboxynaphthyl; cyano (CN) or $-CONH_2$;

n is 1 or 2; and

X is an acid anion.

Examples of substituents which may be included in substituted alkyl groups are alkoxy, such as methoxy; phenyl; 2-carboxyphenyl and cyano groups. Examples of suitable substituents for inclusion in a substituted aryl group include methyl and methoxy.

An especially useful 1,2,4-triazolium-3-thiolate is 1,4,5-trimethyl-3-(2-carboxybenzylthio)-1,2,4-triazolium nitrate represented by the formula (Compound A):

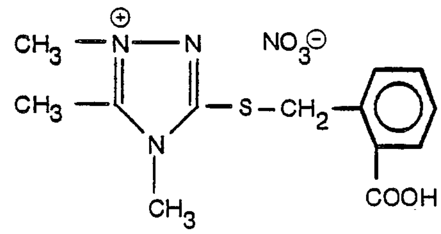

Examples of other useful 1,2,4-triazolium-3-thiolate stabilizer precursors include:

1,4,5-Trimethyl-3-(carboxamidomethylthio)-1,2,4-triazolium trifluoroacetate (Compound B) represented by the formula:

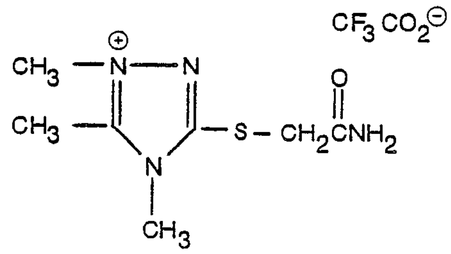

1,5-Dimethyl-3-(carboxamidomethylthio)-4-amino-1,2,4-triazolium trifluoroacetate (Compound C) represented by the formula:

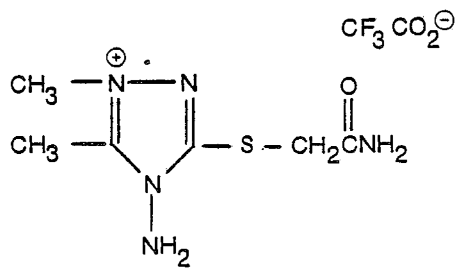

1,5-Dimethyl-3-(cyanomethylthio)-4-amino-1,2,4-triazolium tetrafluoroborate (Compound D) represented by the formula:

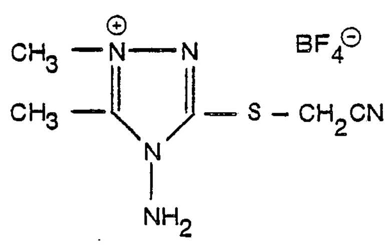

1,4,5-Trimethyl-3-(carboxamidomethylthio)-1,2,4-triazolium dichloroacetate (Compound E) represented by the formula:

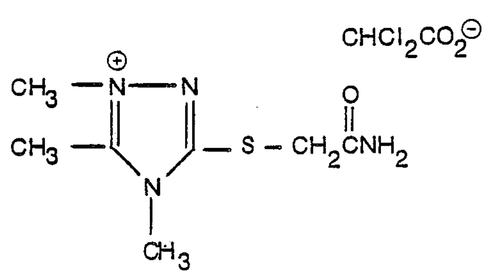

1,5-Dimethyl-3-(carboxamidomethylthio)-4-(2-methoxyethyl)-1,2,4-triazolium nitrate (Compound F) represented by the formula:

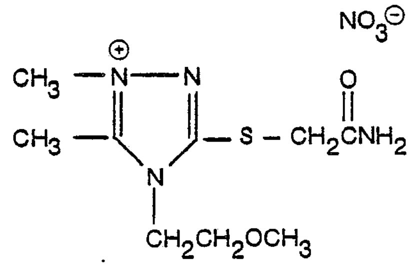

1,4,5-Trimethyl-3-(cyanomethylthio)-1,2,4-triazolium tetrafluoroborate (Compound G) represented by the formula:

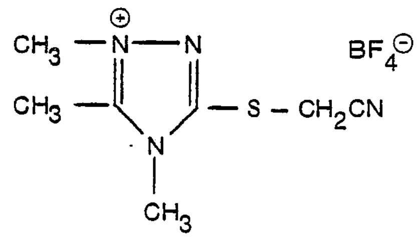

1,4,5-Trimethyl-3-(4-carboxybenzylthio-1,2,4-triazolium nitrate (Compound H) represented by the formula:

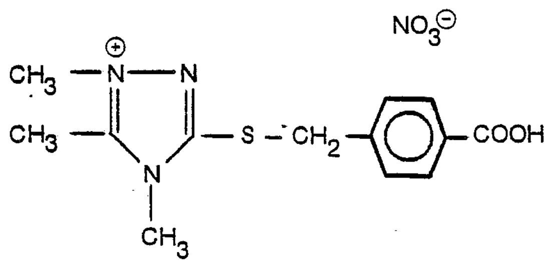

1,4,5-Trimethyl-3-(2-carboxy-6-nitrobenzylthio)-1,2,4-triazolium nitrate (Compound I) represented by the formula:

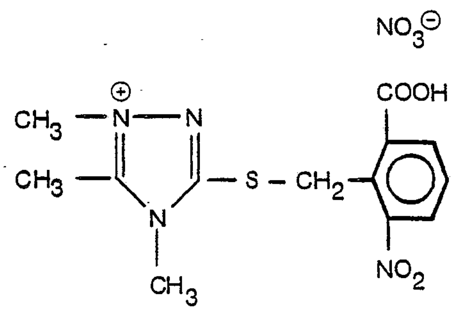

1,4,5-Trimethyl-3-(2-carboxy-3-nitrobenzylthio)-1,2,4-triazolium nitrate (Compound J) represented by the formula:

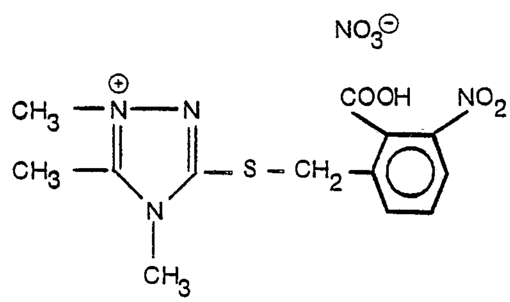

The 1,2,4-triazolium-3-thiolate stabilizer precursor compounds are prepared by methods known in the organic chemical synthesis art. The parent compounds from which the stabilizer precursors are prepared are also prepared by methods known in the chemical art. The preparation of the compound 1,4,5-trimethyl-1,2,4-triazolium-3-thiolate illustrates preparation of a parent compound:

Acetic anhydride (10.2 g, 0.1 mole) was slowly added to a stirred distilled water (11 g) solution of methyl hydrazine (4.6 g, 0.1 mole) at 0°C. The resulting solution was stirred at room temperature for 1 hour and the water was removed under reduced pressure. The residual oily acethydrazide, $CH_3N(COCH_3)NH_2$, was suspended in diethyl ether and to this stirred mixture at room temperature was slowly added a diethyl ether (25 ml) solution of methyl isothiocyanate (7.3 g, 0.1 mole). The resulting stirred solution was kept at room temperature for thirty minutes and then the solvent was removed under reduced pressure. The residual colorless solid was triturated with diethyl ether to provide 4.9 grams (30 percent) of the desired thiosemicarbazide (a white powder) having a melting point of 180 to 181°C. The thiosemicarbazide (5.0 g, 0.03 mole) was refluxed in methanol (25 ml) solution for 21 hours. During this reflux period, the thiosemicarbazide completely dissolved in the refluxing methanol and a colorless solid then separated. The solid had a melting point of 258 to 259°C.

Crude acethydrazide prepared from acetic anhydride (10.2 g, 0.1 mole) and methyl hydrazine (4.6 g, 0.1 mole) as described above, was dissolved in diethyl ether (25 ml) and to the resulting stirred translucent solution at room temperature was slowly added a diethyl ether (25 ml) solution of 2-methoxyethyl isothiocyanate (11.7 g, 0.1 mole). After keeping the stirred solution at ambient temperature for one hour, the ether was removed under reduced pressure. More diethyl ether was added to the residual pale yellow syrup, and the resulting composition was stirred at ambient temperature for 18 hours. This provided a product having a melting point of 122 to 123°C. The thiosemicarbazide (2.4 g, 0.012 mole) was heated to its melting point (above 123°C) and held at this temperature for 5 hours. After cooling to ambient temperature, the crystalline residue was recrystallized from ethyl acetate/ethanol (1:1 by volume) to provide 1.3 grams (59 percent) of pale yellow plates having a melting point of 125 to 126°C. The structure of the desired product was confirmed by mass spectral analysis and nuclear magnetic resonance.

An illustration of a different method of preparation of a parent 1,2,4-triazolium-3-thiolate is the preparation of 1,5-dimethyl-4-(2-methoxyethyl)-1,2,4-triazolium-3-thiolate:

A stirred dichloromethane (40 ml) mixture of 2-methoxyethylisocyanide dichloride (1.9 g, 0.012 mole) and $CH_3—CS—N(CH_3)NH_2$ (1.3 g, 0.012 mole) was refluxed for two hours. The solvent was then removed under reduced pressure and the residual orange semi-solid was dissolved in 50 ml of methanol. One-half of this solution was evaporated to dryness and the residue was then dissolved in methylene chloride (50 ml). Ammonia gas was bubbled through this stirred solution at ambient temperature for about five minutes. The resulting precipitate (presumably ammonium chloride) was collected and the filtrate was evaporated to dryness to yield a reddish-brown semi-solid. A stirred ethanol (50 ml) solution of this solid was refluxed for 18 hours. Solvent was removed under reduced pressure to give a slowly separating orange oil. An ethyl acetate solution of this material was treated with decolorizing carbon and was eluted through a diatomaceous $SiO_2$ absorptive filter material (Celite)®. The ethyl acetate eluate was concentrated to about 25 ml and colorless plates separated. The desired compound was recovered and had a melting point of 123 to 125°C. The product was identified by nuclear magnetic resonance and mass spectral analysis.

The preparation of 1,4,5-trimethyl-3-(2-carboxybenzylthio)-1,2,4-triazolium nitrate illustrates preparation of a 1,2,4-triazolium-3-thiolate stabilizer precursor according to the invention:

A stirred methanol (75 ml) solution of 1,4,5-trimethyl-1,2,4-triazolium-3-thiolate (5.7 g, 0.04 mole) and α-bromo-2-toluic acid (8.6 g, 0.04 mole) was refluxed for one hour. The solvent was removed under reduced pressure and the residual syrup was dissolved in about 20 ml of methanol. Diethyl ether was added to this stirred solution until a permanent cloudiness resulted. This mixture was chilled for 18 hours. At the end of this time, an oil, out of which a colourless solid was forming, had separated. This mixture was rapidly stirred at ambient temperature until formation of the colorless solid appeared complete. The resulting snow-white solid was collected, washed with diethyl ether and dried. This produced the desired product which had a melting point of 175 to 176°C and was identified by elemental analysis as well as by nuclear magnetic resonance.

Propylene oxide (1.8 g, 0.03 mole) in methanol (25 ml) was added slowly to a stirred methanol (25 ml) mixture of 1,4,5-trimethyl-3-(2-carboxybenzylthio)-1,2,4-triazolium bromide (2.0 g, 0.006 mole) and 70.7 percent nitric acid (0.9 g, 0.01 mole) at 2°C. The resulting stirred solution was kept at ambient temperature for 18 hours. Solvents were removed under reduced pressure and the residual clear colorless oil was crystallized with methanol/diethylether (1:1) by volume). The resulting product had a melting point of 139 to 140°C and was identified by nuclear magnetic resonance and mass spectral analysis.

The desired products are purified by procedures known in the chemical art, such as by recrystallization.

One embodiment of the invention is a heat developable and heat stabilizable photographic silver halide material comprising a support having thereon a layer which contains, or adjacent layers which together contain:

(a) photographic silver halide, preferably as a photographic silver halide gelatino emulsion;
(b) a photographic silver halide developing agent;
(c) a concentration of a thermal base releasing compound sufficient to activate the developing agent upon heating the photographic material;

(d) a stabilizing concentration of a 1,2,4-triazolium-3-thiolate stabilizer precursor compound as defined herein, and

(e) a binder.

The photographic material according to the invention comprises photographic silver halide. Useful photographic silver halides include, for example, silver chloride, silver bromide, silver bromoiodide, silver chlorobromoiodide and mixtures thereof. The grain size of the silver halide ranges from course-grain to fine-grain. The photographic silver halide is prepared by procedures known in the photographic art, as described in, for example, *Research Disclosure,* December 1978, Item No. 17643, and *Research Disclosure,* June 1978, Item No. 17029. The photographic materials according to the invention, if desired, also contain addenda which do not adversely effect the desired properties of the materials, such as antifoggants, tone modifiers, chemical sensitizers, hardeners, matting agents, brighteners, absorbing the filter dyes, development modifiers, spectral senitizers and coating aids, as described in these *Research Disclosure* publications.

Many antifoggants are useful in a photographic material according to the invention. Useful antifoggants are selected from those described in, for example *Research Disclosure,* December 1978, Item No. 17643. Tetraazaindene antifoggants are especially useful. Such tetraazaindene antifoggants are described in, for example, U.S. Patent 2,716,062.

The heat developable and heat stabilizable photographic materials according to the invention contain binders and vehicles, alone or in combination. Suitable vehicle materials include both naturally occurring substances such as protein, for example, gelatin, gelatin derivatives, cellulose derivatives, polysaccharides such as dextrin or gum arabic; and synthetic polymeric materials .such as water-soluble polyvinyl compounds such as poly(vinylpyrrolidone) or acrylamide polymers. The photographic layers and other layers of the materials of the invention such as overcoat layers, interlayers and subbing layers can also contain, alone or in combination with the described vehicles, other synthetic polymeric vehicle compounds, such as dispersed vinyl compounds, such as in latex form, in particularly those which increase the dimensional stability of the photographic materials. Useful binders are also described in the above *Research Disclosure* publications. Selection of an optimum binder depends upon such factors as the processing conditions, the particular components of the photographic material and the desired image.

Many supports are useful for a photographic material according to the invention. Typical supports include those which are resistant to adverse changes in structure and do not adversely affect the sensitometric properties of the described photographic materials at the processing temperatures employed. Typical supports include cellulose ester film, poly(vinyl acetal) film, poly(ethylene terephthalate) film, polycarbonate film and related films and resinous materials, as well as glass, paper, or metal.

The stabilizer precursor according to the invention is in a location in the photographic material which enables the stabiliser precursor upon processing to form a stabilizer moiety and react with the silver halide in the unexposed areas upon processing to form a stable silver (I) salt. The stablizer precursor is useful in one or more layers of a photographic material according to the invention. The stabilizer precursor is preferably in an overcoat layer or in a layer between the support and the layer containing silver halide. It is important that the stabilizer percursor be in a location which enables the desired interaction between the moiety produced upon heating the stabilizer precursor and the silver halide after desired development of the exposed silver halide. The term "in reactive association" is often used to mean that the stabilizer precursor and silver halide are in such locations as to enable such desired interaction.

Many silver halide developing agents are useful according to the invention. Combinations of silver halide developing agents are useful. Useful silver halide developing agents include, for instance, those described in *Research Disclosure*, June 1978, Item No. 17029. Preferred silver halide developing agents are ascorbic acid and 4-hydroxymethyl-4-methyl-1-phenyl-3-pyrazolidone.

Many thermal base releasing compounds are useful in a heat developable and heat stabilizable photographic material according to the invention. The term "thermal base releasing compound" herein means a compound which releases a base, preferably an organic base, when heated to processing temperature in a photographic material according to the invention. The released base activates development of the exposed photographic silver halide at processing temperature. The concentration of the base release agent in the photographic material must be sufficient to release a sufficient amount of base upon heat processing to activate development. The base released also helps stabilization by aiding in deblocking the stabilizer precursor according to the invention. Examples of useful thermal base releasing compounds are described in *Research Disclosure,* June 1978, Item No. 17029 and include guanidinium trichloroacetate, 1,1-dimethyl-1-(2-hydroxypropyl)-amine adipimide, 1-(β-aminoethyl)-2-imidazolidone trichloroacetate, zinc oxide and urea.

The optimum concentration of each of (a) photographic silver halide, (b) photographic silver halide developing agent, (c) thermal base release agent, and (d) stabilizer precursor according to the invention will depend upon such factors as the desired image, processing conditions and particular components and their respective concentration in the heat developable and heat stabilizable photographic material. In a photographic material according to the invention, useful concentrations, per square meter of support, are within the following ranges:

(a) photographic silver halide: $2.5 \times 10^{-3}$ to $1.0 \times 10^{-1}$ moles, preferably $1.0 \times 10^{-2}$ to $3.0 \times 10^{-2}$ moles;

(b) photographic silver halide developing agent: $2.5 \times 10^{-3}$ to $1.0 \times 10^{-1}$ moles, preferably $1.0 \times 10^{-2}$ to $3.0 \times 10^{-2}$ moles;

(c) thermal base releasing agent: $1.15 \times 10^{-3}$ to $5.0 \times 10^{-2}$ moles, preferably $5.0 \times 10^{-3}$ to $1.5 \times 10^{-2}$ moles; and

(d) stabilizer precursor: $2.5 \times 10^{-3}$ to $1.0 \times 10^{-1}$ moles, preferably $1.0 \times 10^{-2}$ to $3.0 \times 10^{-2}$ moles;

per square meter of support.

An especially useful heat developable and heat stabilizable photographic material according to the invention comprises a support having thereon a layer which contains, or adjacent layers which together contain:

(a) photographic silver halide gelatino emulsion;

(b) a photographic silver halide developing agent, such as ascorbic acid, 1-phenyl-3-pyrazolidone or 4-hydroxymethyl-4-methyl-1-phenyl-3-pyrazolidone;

(c) a concentration of a thermal base releasing compound consisting essentially of an ethylene-bis(sulfonylacetic acid) compound sufficient to activate the developing agent upon heating the photographic material;

(d) a stabilizing concentration of a 1,2,4-triazolium-3-thiolate stabilizer percursor consisting essentially of 1,4,5-trimethyl-3-(2-carboxybenzylthio)-1,2,4-triazolium nitrate, and

(e) a gelatino binder.

The stabilizer precursors are useful in photographic silver halide processing compositions. Such processing compositions include silver halide monobaths, stabilizing compositions, fixing compositions, hardeners and other processing compositions that enable the stabilizer precursor according to the invention to form a moiety which produces a silver (I) complex without adversely affecting desired properties of the processing composition and the photographic silver halide material. An example of a silver halide processing composition comprises a silver halide developing agent, a thermal base release agent, and a stabilizer precursor such as 1,4,5-trimethyl-3-(2-carboxybenzylthio)-1,2,4-triazolium nitrate. The processing composition generally comprises a solvent or binder.

Because the described stabilizer precursor compounds provide a moiety which can produce a stable silver (I) complex, no additional silver halide stabilizer is necessary in a photographic material according to the invention.

After exposure of a photograhpic silver halide material according to the invention, an image is developed and stabilized by heating the material to a processing temperature within the range of about 100°C to about 180°C, such as about 130°C to about 140°C, until the image is developed and stabilized. An image is generally developed and stabilized by heating for about one to about 60 seconds, such as about 10 to about 30 seconds. Normal atmospheric conditions of pressure and humidity are preferred for processing.

Various means are useful for heating the exposed photographic silver halide material, including a simple hot plate, iron or rollers, dielectric heating means, heated drum or microwave heating means.

The following examples are included for a further understanding of the invention.

## Example 1

Use of a Stabilizer Precursor

The following composition was coated on a gelatin subbed poly(ethylene terephthalate) film support and dried at room temperature (about 20°C):

| Component | Coverage |
|---|---|
| photographic gelatin | 32.4 mg/dm$^2$ |
| surfactant (Surfactant 10G, trade mark for a para-isononylphenoxy-polyglycidol, available from the Olin Corporation, U.S.A.) | 2.2 mg/dm$^2$ |
| methylsuccinic acid | 14.1 mg/dm$^2$ |
| silver bromoiodide gelatino emulsion (0.14 μm grain, unsensitized, 2.5 mole percent iodide) | 10.8 mg/dm$^2$ |
| 1,4,5-trimethyl-3-(2-carboxybenzylthio)-1,2,4-triazolium nitrate (stabilizer precursor) | 44.3 mg/dm$^2$ |

The melt pH of the composition was adjusted to 4.5 by means of 1N-potassium hydroxide. The resulting photographic material was heated at 140°C for 15 seconds. The heated material was light stable and transparent (specular density of 0.25).

A similar photographic material was used to determine silver halide dissolution (initial) rates, identified as $r_{id}$, at 120°C and 38°C. The ratio R of the $r_{id}$'s at these two temperatures was a measure of the activation energy for silver halide dissolution according to the following equation:

$$R_d = \frac{r_{id} \text{ at } 120°C}{r_{id} \text{ at } 38°C}$$

From the incubation stability point of view, a compound was considered to be useful as a stabilizer precursor if its $R_d$ value was larger than $10^3$. The $R_d$ value for the stabilizer precursor of this example was 2.0 times $10^4$.

Example 2

The following composition was prepared and coated on a gelatin subbed poly(ethylene terephthalate) film support at a wet coating thickness of 0.01 centimeter:

| Component | Coverage (Amount per $dm^2$) |
|---|---|
| photographic gelatin | 43.2 mg |
| silver chloride gelatino emulsion (0.24 μm grain size, unsensitized) | 0.119 mmole |
| ascorbic acid (silver halide developing agent) | 0.076 mmole |
| maleic acid (buffer) | 0.150 mmole |
| $[H_2N—(CH_2)_3—NHCO—NH—CH_2]_2—CH_2$ (development modifier) | 0.032 mmole |
| 4-hydroxy-6-methyl-1,3,3a,7-tetraazaindene (antifoggant) | 1.1 mg |
| 1,4,5-trimethyl-3-(2-carboxybenzylthio)-1,2,4-triazolium nitrate (stabilizer precursor) | 0.161 mmole |
| surfactant (Surfactant 10G) | 1.1 mg |

The melt pH for the described composition was adjusted to 4.5 by means of 1N-potassium hydroxide. The resulting photographic material was permitted to dry and then was overcoated with 540 mg per square metre of polymethyl methacrylate from a dichloromethane solution. This produced a heat developable and heat stabilizable photographic material according to the invention.

The photographic material was sensitometrically exposed to white light in a commercial sensitometer and then heated for 15 seconds 140°C. A developed and stabilized image was produced having a maximum density (diffused) of 1.5 and a minimum density of 0.6.

Example 3

Compound identified below in Table I can be used to obtain a developed and stabilized image when coated and processed in a manner similar to that reported above in Example 2:

TABLE I

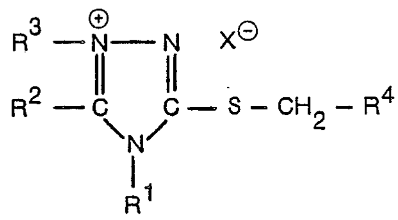

STABILIZER PRECURSOR

| Example | $R^3$ | $R^4$ | $R^1$ | $R^2$ | X |
|---|---|---|---|---|---|
| 3(a) | $CH_3$ | $CONH_2$ | $CH_3$ | $CH_3$ | $CF_3CO_2$ |
| 3(b) | $CH_3$ | $C(CH_3)_3$ | $CH_3$ | $CH_3$ | $NO_3$ |
| 3(c) | $CH_3$ | $CONH_2$ | $NH_2$ | $CH_3$ | $CF_4CO_2$ |
| 3(d) | $CH_3$ | phenyl | $NH_2$ | $CH_3$ | $NO_3$ |
| 3(e) | $CH_3$ | CN | $NH_2$ | $CH_3$ | $BF_4$ |
| 3(f) | i-$C_3H_7$ | $CONH_2$ | $CH_3$ | i-$C_3H_7$ | $BF_4$ |
| 3(g) | $CH_3$ | $CONH_2$ | $CH_3$ | $CH_3$ | $CHCl_2CO_2$ |
| 3(h) | phenyl | $CONH_2$ | $CH_3$ | $CH_3$ | $BF_4$ |
| 3(i) | $CH_3$ | $CONH_2$ | $CH_2CH_2OCH_3$ | $CH_3$ | $NO_3$ |
| 3(j) | $C_2H_5$ | CN | $CH_3$ | phenyl | $BF_4$ |
| 3(k) | $CH_3$ | CN | $CH_3$ | $CH_3$ | $BF_4$ |
| 3(l) | $CH_3$ | $CONH_2$ | phenyl | $CH_3$ | $BF_4$ |
| 3(m) | $CH_3$ | $CONH_2$ | benzyl | $CH_3$ | $BF_4$ |
| 3(n) | $CH_3$ | 4-COOH-phenyl | $CH_3$ | $CH_3$ | $NO_3$ |
| 3(o) | $CH_3$ | 2-COOH-6-$NO_2$phenyl | $CH_3$ | $CH_3$ | $NO_3$ |
| 3(p) | $CH_3$ | 2-COCH-3-$NO_2$phenyl | $CH_3$ | $CH_3$ | $NO_3$ |
| 3(q) | $CH_3$ | 2-COOH-phenyl | cyclohexyl | $CH_3$ | $NO_3$ |
| 3(r) | $CH_3$ | 2-COOH-phenyl | $C_{18}H_{37}$ | $CH_3$ | $NO_3$ |
| 3(s) | $CH_3$ | 2-COOH-phenyl | $CH_2CHCH_2$ | $CH_3$ | $NO_3$ |
| 3(t) | $CH_3$ | $CH_2CONH_2$ | $CH_3$ | $CH_3$ | $NO_3$ |

**Claims**

1. A heat developable and heat stabilizable photographic silver halide material comprising a support having thereon a layer which contains, or adjacent layers which together contain,

(a) photographic silver halide,

(b) a photographic silver halide developing agent,

(c) a concentration of a thermal base releasing compound sufficient to activate the developing agent upon heating the photographic material;

(d) a silver halide stabilizer precursor compound and

(e) a binder;

characterized in that said stabilizer precursor compound is a 1,2,4-triazolium-3-thiolate containing a heat releasable blocking group on the sulfur atom.

2. A photographic material according to Claim 1 characterized in that said 1,2,4-triazolium-3-thiolate compound has the formula:

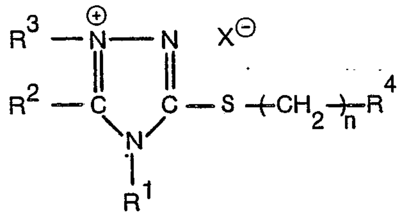

wherein:

$R^1$ is substituted or unsubstituted alkyl containing 1 to 18 carbon atoms, $NR^5R^6$ wherein $R^5$ and $R^6$ can be hydrogen, substituted or unsubstituted alkyl of from 1 to 18 carbon atoms or substituted or unsubstituted aryl of from 6 to 20 carbon atoms, with the proviso that when $R^5$ is alkyl, $R^6$ is also alkyl; alkenyl containing 3 to 18 carbon atoms; cycloalkyl containing 3 to 12 carbon atoms; or substituted unsubstituted aryl containing 6 to 20 carbon atoms;

$R^2$ is substituted or unsubstituted alkyl containing 1 to 9 carbon atoms; or aryl containing 6 to 12 carbon atoms;

$R^3$ is substituted or unsubstituted alkyl containing 1 to 18 carbon atoms; or substituted or unsubstituted aryl containing 6 to 12 carbon atoms, or cycloalkyl of from 3 to 12 carbon atoms; and

$R^4$ is substituted or unsubstituted alkyl containing 1 to 18 carbon atoms, substituted or unsubstituted aryl containing 6 to 12 carbon atoms; carboxyaryl containing 7 to 13 carbon atoms; cyano (CN) or $-CONH_2$;

n is 1 or 2; and

X is an acid anion.

3. A photographic material according to Claims 1 or 2 characterized in that said 1,2,4-trizolium-3-thiolate compound is 1,4,5-trimethyl-3-(2-carboxybenzylthio)-1,2,4-triazolium nitrate, represented by the formula:

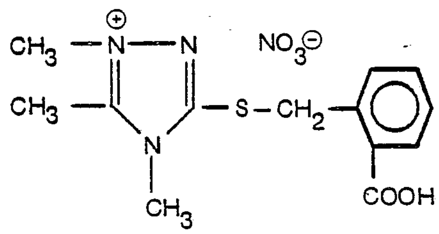

1,4,5-trimethyl-3-(cyanomethylthio)-1,2,4-triazolium tetrafluoroborate represented by the formula:

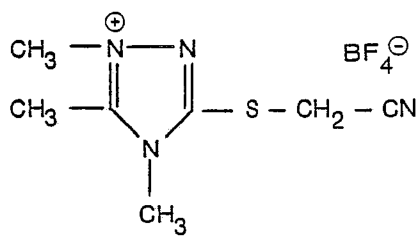

4. A heat activatable photographic silver halide processing composition comprising a photographic silver halide developing agent, a thermal base releasing compound and a stabilizing concentration of silver halide stabilizer precursor compound characterized in that said stabilizer percursor compound is a 1,2,4-triazolium-3-thiolate stabilizer precursor compound containing a heat releasable blocking group on the sulfur atom.

5. A processing composition according to claim 4 characterized in that said stabilizer precursor is 1,4,5-trimethyl-3-(2-carboxybenzylthio)1,2,4-triazolium nitrate, 1,4,5-trimethyl-3-cyanomethylthio-1,2,4-triazolium tetrafluoroborate or a combination thereof.

6. A 1,2,4-triazoluim-3-thiolate precursor compound represented by the formula:

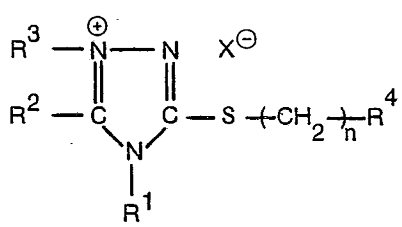

wherein:

$R^1$ is substituted or unsubstituted alkyl containing 1 to 18 carbon atoms, $NR^5R^6$ wherein $R^5$ and $R^6$ can be hydrogen, substituted or unsubstituted alkyl of from 1 to 18 carbon atoms or substituted or unsubstituted aryl of from 6 to 20 carbon atoms, with the proviso that when $R^5$ is alkyl, $R^6$ is also alkyl; alkenyl containing 3 to 18 carbon atoms; cycloalkyl containing 3 to 12 carbon atoms, or substituted unsubstituted aryl containing 6 to 20 carbon atoms;

$R^2$ is substituted or unsubstituted alkyl containing 1 to 9 carbon atoms; or aryl containing 6 to 12 carbon atoms;

$R^3$ is substituted or unsubstituted alkyl containing 1 to 18 carbon atoms; or substituted or unsubstituted aryl containing 6 to 12 carbon atoms; or cycloalkyl of from 3 to 12 carbon atoms; and

$R^4$ is substituted or unsubstituted alkyl containing 1 to 18 carbon atoms; substituted or unsubstituted aryl containing 6 to 12 carbon atoms; carboxyaryl containing 7 to 13 carbon atoms; cyano .(CN) or $-CONH_2$;

n is 1 or 2; and

X is an acid anion.

7. 1,4,5-Trimethyl-3-(2-carboxybenzylthio)-1,2,4-triazolium nitrate represented by the formula:

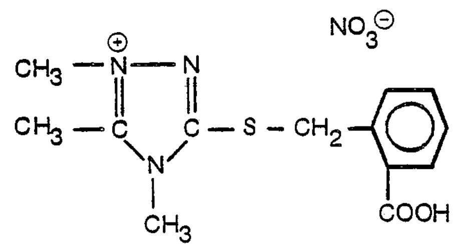

8. 1,4,5-Trimethyl-3-(cyanomethylthio)-1,2,4-triazolium tetrafluoroborate represented by the formula:

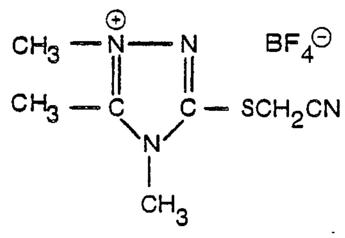

9. 1,4,5-Trimethyl-3-(carboxamidomethylthio)-1,2,4-triazolium trifluoroacetate represented by the formula:

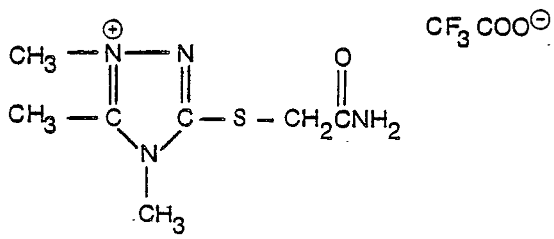

**Revendications**

1. Produit photographique contenant de l'halogénure d'argent développable et stabilisable par la chaleur, comprenant un support portant une couche qui contient les composés suivants, ou des couches contiguës qui, ensemble, contiennent ces composés:

a) l'halogénure d'argent photographique,

b) un agent développateur de l'halogénure d'argent photographique,

c) un composé qui libère une base sous l'action de la chaleur, en concentration suffisante pour activer l'agent développateur lors du chauffage du produit photographique,

d) un composé précurseur d'un agent stabilisant de l'halogénure d'argent et

e) en liant,

caractérisé en ce que le composé précurseur de l'agent stabilisant est un 1,2,4-triazolium-3-thiolate comprenant, sur l'atome de soufre, un groupe de blocage libérable par action de la chaleur.

2. Produit photographique conforme à la revendication 1, caractérisé en ce que le composé 1,2,4-triazolium-3-thiolate a la formule:

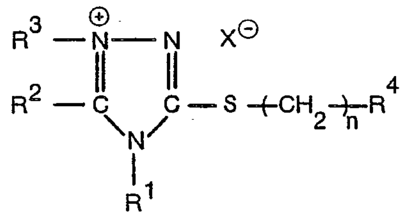

où

$R^1$ est un groupe alkyle substitué ou non, contenant 1 à 18 atomes de carbone, $NR^5R^6$ où $R^5$ et $R^6$ peuvent être un atome d'hydrogène, un groupe alkyle substitué ou non ayant de 1 à 18 atomes de carbone ou un groupe aryle substitué ou non de 6 à 20 atomes de carbone, à la condition que $R^6$ soit un groupe alkyle lorsque $R^5$ est un groupe alkyle; un groupe alkényle contenant 3 à 18 atomes de carbone; un groupe cycloalkyle contenant 3 à 12 atomes de carbone ou un groupe aryle substitué ou non contenant 6 à 20 atomes de carbone;

$R^2$ est un groupe alkyle substitué ou non contenant 1 à 9 atomes de carbone ou un groupe aryle contenant 6 à 12 atomes de carbone;

$R^3$ est un groupe alkyle substitué ou non contenant 1 à 18 atomes de carbone ou un groupe aryl substitué ou non contenant 6 à 12 atomes de carbone ou un groupe cycloalkyle ayant de 3 à 12 atomes de carbone;

$R^4$ est un groupe alkyle substitué ou non contenant 1 à 18 atomes de carbone, un groupe aryle substitué ou non contenant 6 à 12 atomes de carbone, un groupe carboxyaryle contenant 7 à 13 atomes de carbone, un groupe cyano (CN) ou un groupe $—CONH_2$;

n est égal à 1 ou 2, et

X est un anion acide.

3. Produit photographique conforme aux revendications 1 ou 2, caractérisé en ce que le composé 1,2,4-triazolium-3-thiolate est le nitrate de 1,4,5-triméthyl-3-(2-carboxybenzylthio)-1,2,4-triazolium, représenté par la formule:

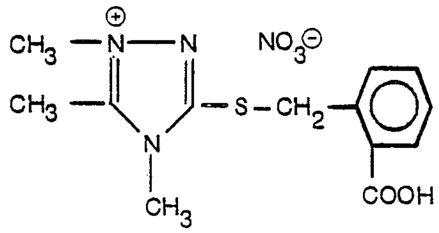

ou

le tétrafluoroborate de 1,4,5-triméthyl-3-(cyanométhylthio)-1,2,4-triazolium représenté par la formule:

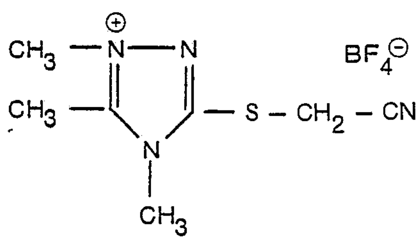

4. Composition de traitement des halogénures d'argent photographiques, activable par la chaleur, comprénant un agent développateur des halogénures d'argent photographiques, un composé libérant une base sous l'action de la chaleur et un composé, précurseur d'un agent stabilisant des halogénures d'argent, utilisé en concentration permettant de stabiliser, caractérisée en ce que le composé précurseur de l'agent stabilisant est un 1,2,4-triazolium-3-thiolate comprenant, sur l'atome de soufre, un groupe de blocage libérable par action de la chaleur.

5. Composition de traitement, conforme à la revendication 4, caractérisé en ce que le précurseur de l'agent stabilisant est le nitrate de 1,4,5-triméthyl-3-(2-carboxybenzylthio)-1,2,4-triazolium, le tètrafluoroborate de 1,4,5-triméthyl-3-cyano-méthylthio-1,2,4-triazolium ou un mélange de ces composés.

6. Composé précurseur 1,2,4-triazolium-3-thiolate, représenté par la formule:

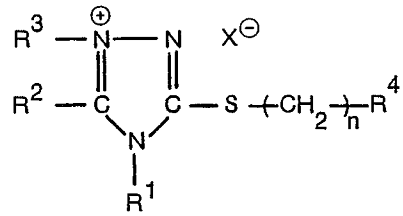

où

$R^1$ est un groupe alkyle substitué ou non, contenant 1 à 18 atomes de carbone, $NR^5R^6$ où $R^5$ et $R^6$ peuvent être un atome d'hydrogène, un groupe alkyle substitué ou non ayant de 1 à 18 atomes de carbone ou un groupe aryle substitué ou non de 6 à 20 atomes de carbone, à la condition que $R^6$ soit un groupe alkyle lorsque $R^5$ est un groupe alkyle; un groupe alkényle contenant 3 à 18 atomes de carbone, un groupe cycloalkyle contenant 3 à 12 atomes de carbone ou un groupe aryle substitué ou non contenant 6 à 20 atomes de carbone;

$R^2$ est un groupe alkyle substitué ou non contenant 1 à 9 atomes de carbone ou un groupe aryle contenant 6 à 12 atomes de carbone;

$R^3$ est un groupe alkyle substitué ou non contenant 1 à 18 atomes de carbone ou un groupe aryle substitué ou non contenant 6 à 12 atomes de carbone ou un groupe cylcloalkyle ayant de 3 à 12 atomes de carbone;

$R^4$ est un groupe alkyle substitué ou non contenant 1 à 18 atomes de carbone, un groupe aryle substitué ou non contenant 6 à 12 atomes de carbone, un groupe carboxyaryle contenant 7 à 13 atomes de carbone, un groupe cyano (CN) ou un groupe $—CONH_2$;

n est égal à 1 ou 2; et

X est un anion acide.

7. Nitrate de 1,4,5-triméthyl-3-(2-carboxybenzylthio)-1,2,4-triazolium représenté par la formule:

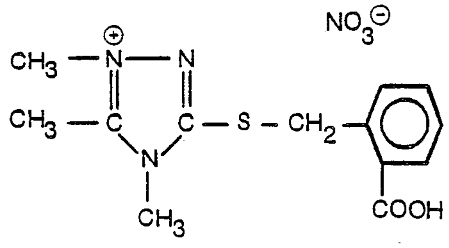

8. Tétrafluoroborate de 1,4,5-triméthyl-3-(cyanométhylthio)-1,2,4-triazolium représenté par la formule:

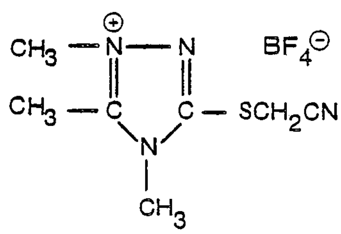

9. Trifluoroacétate de 1,4,5-triméthyl-3-(carboxamidométhylthio)-1,2,4-triazolium représenté par la formule:

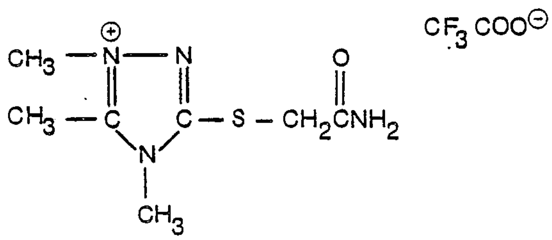

**Patentansprüche**

1. Durch Einwirkung von Wärme entwickelbares und durch Wärme stabilisierbares photographisches Silberhalogenidmaterial mit einem Schichtträger und einer hierauf angeordneten Schicht, die enthält oder benachbarten Schichten, die zusammen enthalten:

(a) photographisches Silberhalogenid,
(b) eine photographische Silberhalogenid-Entwicklerverbindung,
(c) eine bei Einwirkung von Wärme eine Base freisetzende Verbindung in einer Konzentration, die ausreicht, um die Entwicklerverbindung bei Erwärmung des photographischen Materials zu aktivieren,
(d) eine Silberhalogenid-Stabilisatorvorläuferverbindung und
(e) ein Bindemittel,

dadurch gekennzeichnet, daß die Stabilisatorvorläuferverbindung ein 1,2,4-Triazolium-3-thiolat mit einer bei Einwirkung von Wärme freisetzbaren blockierenden Gruppe am Schwefelatom ist.

2. Photographisches Material nach Anspruch 1, dadurch gekennzeichnet, daß die 1,2,4-Triazolium-3-thiolatverbindung der folgenden Formel entspricht:

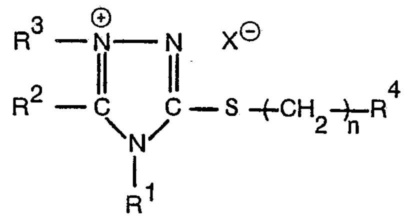

in der bedeuten:

$R^1$ eine substituierte oder nicht-substituierte Alkylgruppe mit 1 bis 18 C-Atomen, einen Rest der Formel $-NR^5R^6$, worin $R^5$ und $R^6$ Wasserstoffatome, substituierte oder nicht-substituierte Alkylgruppen mit 1 bis 18 C-Atomen oder substituiete oder nicht-substituierte Arylgruppen mit 6 bis 20 C-Atomen darstellen, wobei gilt, daß wenn $R^5$ für eine Alkylgruppe steht, $R^6$ ebenfalls eine Alkylgruppe ist; eine Alkenylgruppe mit 3 bis 18 C-Atomen; eine Cycloalkylgruppe mit 3 bis 12 C-Atomen oder eine substituierte oder nicht-substituierte Arylgruppe mit 6 bis 20 C-Atomen;

$R^2$ eine substituierte oder nicht-substituierte Alkylgruppe mit 1 bis 9 C-Atomen oder eine Aryl-gruppe mit 6 bis 12 C-Atomen;

$R^3$ eine substituierte oder nicht-substituierte Alkylgruppe mit 1 bis 18 C-Atomen oder eine substituierte oder nicht-substituierte Arylgruppe mit 6 bis 12 C-Atomen oder eine Cycloalkylgruppe mit 3 bis 12 C-Atomen und

$R^4$ eine substituierte oder nicht-substituierte Alkylgruppe mit 1 bis 18 C-Atomen, eine substituierte oder nicht-substituierte Arylgruppe mit 6 bis 12 C-Atomen, eine Carboxyarylgruppe mit 7 bis 13 C-Atomen, eine Cyanogruppe ($-CN$) oder $-CONH_2$;

n = 1 oder 2 und

X ein Säure anion.

3. Photographisches Material nach Ansprüchen 1 oder 2, dadurch gekennzeichnet, daß die 1,2,4-Triazolium-3-thiolatverbindung 1,4,5-Trimethyl-3-(2-carboxybenzylthio)-1,2,4-triazoliumnitrat der Formel:

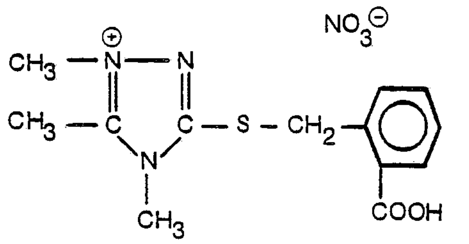

oder

1,4,5-Trimethyl-3-(cyanomethylthio)-1,2,4-triazoliumtetrafluoroborat der Formel:

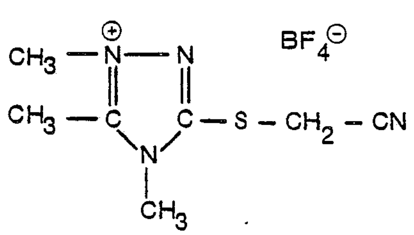

ist.

4. Durch Einwirkung von Wärme aktivierbare photographische Silberhalogenid-Entwicklermasse mit einer photographischen Silberhalogenidentwicklerverbindung, einer bei Einwirkung von Wärme eine Base freisetzenden Verbindung und einer Silberhalonid-Stabilisatorvorläuferverbindung in stabilisierender Konzentration, dadurch gekennzeichnet, daß die Stabilisatorvorläuferbindung eine 1,2,4-Triazolium-3-thiolat-Stabilisatorvorläuferverbindung ist, die am Schwefelatom eine durch Einwirkung von Wärme freisetzbare blockierende Gruppe aufweist.

5. Entwicklermasse nach Anspruch 4, dadurch gekennzeichnet, daß die Stabilisatorvorläuferverbindung 1,4,5-Trimethyl-3-(2-carboxybenzylthio)1,2,4-triazoliumnitrat, 1,4,5-Trimethyl-3-cyanomethylthio-1,2,4-triazoliumtetrafluoroborat oder eine Kombination hiervon ist.

6. Eine 1,2,4-Triazolium-3-thiolat-Vorläuferverbindung der Formel:

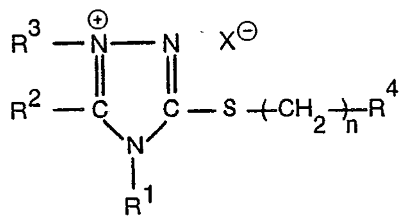

in der bedeuten:

$R^1$ eine substituierte oder nicht-substituierte Alkylgruppe mit 1 bis 18 C-Atomen einen Rest der Formel $—NR^5R^6$, worin $R^5$ und $R^6$ Wasserstoffatome, substituierte oder nicht-substituierte Alkylgruppen mit 1 bis 18 C-Atomen oder substituierte oder nicht-substituierte Arylgruppen mit 6 bis 20 C-Atomen darstellen, wobei gilt, daß wenn $R^5$ für eine Alkylgruppe steht, $R^6$ ebenfalls eine Alkylgruppe ist; eine Alkenylgruppe mit 3 bis 18 C-Atomen, eine Cycloalkylgruppe mit 3 bis 12 C-Atomen oder eine substituierte oder nicht-substituierte Arylgruppe mit 6 bis 20 C-Atomen;

$R^2$ eine substituierte oer nicht-substituierte Alkylgruppe mit 1 bis 9 C-Atomen oder eine Arylgruppe mit 6 bis 12 C-Atomen;

$R^3$ eine substituierte oder nicht-substituierte Alkylgruppe mit 1 bis 18 C-Atomen oder eine substituierte oder nicht-substituierte Arylgruppe mit 6 bis 12 C-Atomen oder eine Cycloalkylgruppe mit 3 bis 12 C-Atomen und

$R^4$ eine substituierte oder nicht-substituierte Alkylgruppe mit 1 bis 18 C-Atomen, eine substituierte oder nicht-substituierte Arylgruppe mit 6 bis 12 C-Atomen, eine Carboxyarylgruppe mit 7 bis 13 C-Atomen, eine Cyanogruppe (—CN) oder $—CONH_2$,

n = 1 oder 2 und

X ein Säureanion.

7. 1,4,5-Trimethyl-3-(2-carboxybenzylthio)-1,2,4-triazoliumnitrat der Formel

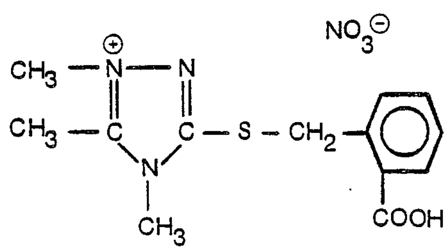

8. 1,4,5-Trimethyl-3-(cyanomethylthio)-1,2,4-triazoliumtetrafluoroborat der Formel:

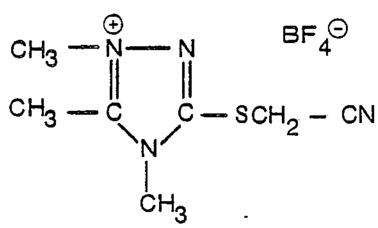

9. 1,4,5-Trimethyl-3-(carboxamidomethylthio)-1,2,4-triazoliumtrifluoroacetat der Formel: